(19) Europäisches Patentamt
European Patent Office
Office européen des brevets

(11) Publication number: **0 282 050 B1**

(12) **EUROPEAN PATENT SPECIFICATION**

(45) Date of publication of patent specification: **06.10.93**

(51) Int. Cl.[5]: **C07D 211/90**, C07D 401/12, A61K 31/44

(21) Application number: **88103776.6**

(22) Date of filing: **10.03.88**

The file contains technical information submitted after the application was filed and not included in this specification

# (54) Carbonyl- and sulphonyl- derivatives of 2-(aminoalkylthio)- methyl-1,4-dihydropyridines, a method for their preparation and pharmaceutical compositions containing them.

(30) Priority: **12.03.87 IT 1965887**

(43) Date of publication of application:
**14.09.88 Bulletin 88/37**

(45) Publication of the grant of the patent:
**06.10.93 Bulletin 93/40**

(84) Designated Contracting States:
**AT BE CH DE ES FR GB GR IT LI LU NL SE**

(56) References cited:
**EP-A- 0 089 167**
**EP-A- 0 119 050**
**EP-A- 0 200 524**
**EP-A- 0 206 747**
**WO-A-87/00836**

**Progr. Pharmacol. 5, 25 ( 1982)**

**Nature 303, 535 ( 1983 )**

**Drug Res. 33, 1268 ( 1983 )**

(73) Proprietor: **BOEHRINGER MANNHEIM ITALIA S.P.A.**
**Via S. Uguzzone, 5**
**I-20126 Milano(IT)**

(72) Inventor: **Frigerio, Marco**
**Via Sant' Uguzzone 5**
**I-20126 Milano(IT)**
Inventor: **Riva, Carlo**
**Via Sant' Uguzzone 5**
**I-20126 Milano(IT)**
Inventor: **Gandolfi, Carmelo A.**
**Via Sant' Uguzzone 5**
**I-20126 Milano(IT)**
Inventor: **Tofanetti, Odoardo**
**Via Sant' Uguzzone 5**
**I-20126 Milano(IT)**
Inventor: **Tognella, Sergio**
**Via Sant' Uguzzone 5**
**I-20126 Milano(IT)**

(74) Representative: **Weber, Manfred, Dr. et al**
**c/o Boehringer Mannheim GmbH,**
**Patentabteilung,**
**Sandhoferstrasse 116**
**D-68298 Mannheim (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention refers to carbonyl and sulphonyl derivatives of 2-(aminoalkylthio)methyl-1,4-dihydropyridines, a method for their preparation and pharmaceutical compositions containing them.

The compounds of the present invention have the following general formula I

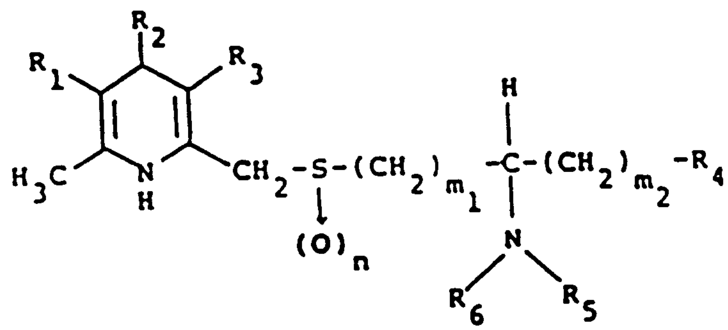

(I)

wherein

- $R_1$ is a C1-C3-alkoxycarbonyl group
- $R_2$ is a m-nitrophenyl group;
- $R_3$ is a $COOR_7$ group;
- $R_4$ is hydrogen or methyl;
- $R_5$ is hydrogen or butyl;
- $R_6$ is a

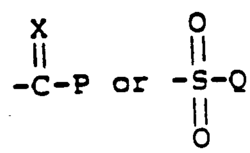

group
wherein X is oxygen or sulphur; P is $C_1$-$C_5$-alkylthio, benzylthio, $C_1$-$C_5$-alkoxy, benzyloxy group and an amino group

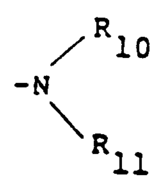

wherein $R_{10}$ and $R_{11}$, that can be the same or different, are hydrogen, $C_1$-$C_5$-alkyl or, taken together with the nitrogen atom, form a piperazine, 4-methyl-piperazine, or 4-(2-hydroxyethyl)piperazine ring; Q is $C_1$-$C_5$-alkyl, phenyl or benzyl;
- $R_7$ is a $C_1$-$C_6$ alkyl group,
- n is 0, 1 or 2; $m_1$ is 1 or 2 and $m_2$ is 0 or 1

their optical isomers, diastereoisomers and pharmaceutically acceptable salts.

Also included in the scope of the present invention are the pharmaceutically acceptable salts, optical antipodes, i.e. the single enantiomers; racemic mixtures of optical antipodes, diasteroisomers of compounds I and mixtures thereof.

Pharmaceutically acceptable salts are those with non toxic acids and bases.

Preferred compounds of the invention are those wherein the $-(CH_2)m_2-R_4$ group is hydrogen or methyl ($m_2$ = 0 or 1 and $R_4$ = hydrogen or methyl); $R_5$ is hydrogen or butyl and $m_1$ is 1 or 2.

Alkyl groups of compounds of the invention may have both linear or branched chain.

A $C_1$-$C_6$-alkyl group is preferably methyl, ethyl, isopropyl or ter-butyl.

In EP-A-200 524 and EP-A-206 747 are disclosed 1,4-dihydropyridines with a similar side chain in position 2, in which the terminal amino group is substituted by carbonyl, thiocarbonyl or sulfonyl residues. In EP-A-89 162 and EP-A-119 050 are described 1,4-dihydripyridines with an oxymethyl side chain in position 2 instead of a thiomethyl group.
All prior art compounds possess an antihypertensive activity but with a relative high toxicity.

Specific examples of compounds of the invention are shown in Table I.

Table I

| N° | $R_1$ | $R_2$ | $R_3$ | $R_4$ | $R_5$ | $R_6$ | $m_1$ | $m_2$ | n |
|---|---|---|---|---|---|---|---|---|---|
| 1 | $CO_2Me$ | $m-NO_2C_6H_4$ | $CO_2Et$ | H | H | $-C(=O)-NH_2$ | 1 | 0 | 0 |
| 2 | $CO_2Me$ | $m-NO_2C_6H_4$ | $CO_2Et$ | H | H | $-C(=S)NHCH_3$ | 1 | 0 | 0 |
| 3 | $CO_2Et$ | $m-NO_2C_6H_4$ | $CO_2Et$ | H | H | $-C(=O)-NH_2$ | 1 | 1 | 0 |
| 4 | $CO_2Et$ | $m-NO_2C_6H_4$ | $CO_2Et$ | H | H | 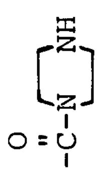 | 1 | 0 | 0 |
| 5 | $CO_2Et$ | $m-NO_2C_6H_4$ | $CO_2Et$ | H | H | 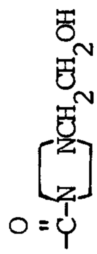 | 1 | 0 | 0 |
| 6 | $CO_2Et$ | $m-NO_2C_6H_4$ | $CO_2ET$ | $CH_3$ | H | $-C(=O)-OEt$ | 1 | 0 | 0 |
| 7 | $CO_2Me$ | $m-NO_2C_6H_4$ | $CO_2Et$ | H | H | $-C(=O)-SC_2H_5$ | 1 | 0 | 0 |

The compounds of the invention are prepared by means of a process including the reaction of a 2-(aminoalkylthiomethyl)-1,4-dihydropyridine of formula II

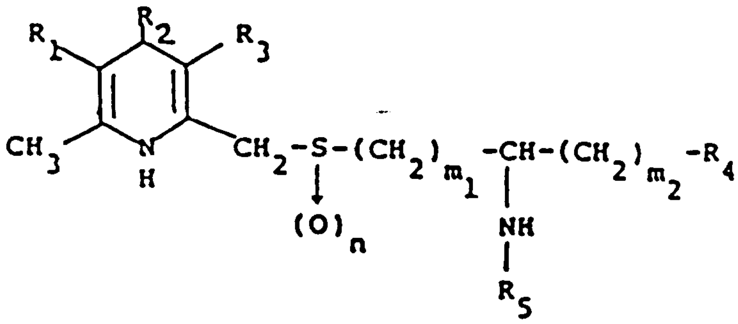

(II)

wherein $R_1$, $R_2$, $R_3$, $R_4$, $R_5$, n, $m_1$, $m_2$ are as above defined with an acylating agent as an activated form of an ester or an amide of a carbonic or thiocarbonic acid of formula III or an activated form of a sulphonic acid of formula IV

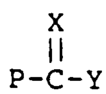

(III)

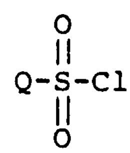

(IV)

wherein P, Q and X are as above defined and Y is selected in the group consisting of chlorine and 1-imidazolyl, in the presence or not of a base to give a compound of formula I.

The reaction of a compound of formula II with the acylating agent of formulae III or IV may be carried out using equimolecular amounts or a slight excess of the acylating species.

Suitable solvents are halogenated hydrocarbons such as $CH_2Cl_2$, 1,2-dichlorethane, chloroform, aliphatic hydrocarbons such as hexane, heptane; cycloaliphatic hydrocarbons such as cyclohexane; aromatic hydrocarbons such as benzene, toluene, pyridine, collidine; linear or cylic ethers such as tetrahydrofurane, diethylether, dimethoxyethane; amides and esters such as dimethylformamide; ethyl acetate; and mixtures thereof.

If, during the reaction, a development of halogenhydric acid occurs (for instance when compounds IV or III, wherein Y is a halogen atom, are used) it is necessary to add stoichiometrical quantities of a base. This base can be an inorganic one such as an alkaline or alkaline-earth carbonate or bicarbonate, or an organic base such as a tertiary amine, e.g. triethylamine, pyridine, an alkyl-substituted pyridine, 4-diethylaminopyridine, etc.

The reaction is preferably carried out at temperatures ranging from -25 °C to the reflux temperature of the solvent, but preferably at room temperature.

Reaction times may vary from few minutes to several days, but usually they do not exceed two hours and a few minutes are often enough to complete the reaction.

The optional oxidation of a compound of formula I, wherein n is 0, to give a compound of formula I, wherein n is 1 or 2, is carried out by treatment with a solution of an inorganic or an organic peracid, for instance a solution of monoperphtalic acid in ethyl acetate. To obtain compounds I wherein n is 1, a molar equivalent of the oxidizing agent is used, while compounds wherein n is 2 are obtained by using an excess of the oxidizing agent (preferably 2 molar equivalents).

Other oxidizing agents may be used, such as a m-chloroperbenzoic, performic, peracetic, peroxytrifluoroacetic, periodic acid or hydrogen peroxide.

When the reaction is over, that is generally carried out from 0 °C to room temperature and for a time ranging from some minutes to few hours, the excess reagent is destroyed by an excess of reducing agent, such as sodium bisulphite, sodium thiosulphate, potassium iodide, in accordance with well-known methods.

The oxidation reaction of compounds of I may be also carried out using as an oxidating agent sodium periodate in alcohol solution (methyl, ethyl, propyl alcohol) at a temperature ranging from 0 °C to the solvent's boiling temperature, for a time ranging from few minutes to some hours.

The preferred temperature is the room temperature and the reaction time is ranging from 1 to 4 hours.

The optional saponification of the $COOR_7$ ester functions is carried out by reaction with diluted aqueous solutions of alkaline hydrates or carbonates such as lithium hydrate, sodium and potassium bicarbonates; ter-butyl esters are removed by treatment with acids, benzyl esters are removed by transfer-hydrogenation with alkaline hypophosphites in the presence of water and Pd on carbon in alcohol solvents such as ethanol, propanol or in acids such as acetic and propionic acids.

The compounds of the invention wherein one of $R_1$ and $R_3$ is a free carboxy group may be subjected to salification with pharmaceutically acceptable bases, or to optical separation with optically active bases, such as for instance (+) and (-) ephedrine, (+) and (-)phenylethylamine, dehydroabiethylamine, according to known methods.

Similarly, the compounds of formula I wherein basic functional groups are present may be salified with pharmaceutically acceptable acids. If said acids are optically active, for example tartaric, mandelic, malic, camphorsulphohic acids, the salts may be subjected to optical resolution, thus obtaining the single optical antipodes.

Compounds of formula III wherein Y is halogen are known compounds commercially available and/or prepared with known methods by monoesterification of phosgene and thiophosgene with alcohols, thiols, primary and secondary amines, as it is well known to the experts in the art.

The compounds of formula III wherein Y is 1-imidazolyl are prepared "in situ" by reaction of a molar equivalent of a diimidazolide of formula V

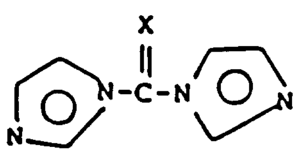

(V)

wherein X is O or S, with a molar equivalent of a suitable alcohol, thiol or an amine of formula VI

P — H (VI)

wherein P is as above defined.

The diimidazolides V are known non toxic synthetic equivalents of phosgene and thiophosgene.

An alternative process for the preparation of compounds I wherein $R_6$ is

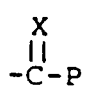

includes the reaction of a compound of formula II with a diimidazolide of formula V, thus obtaining an imidazolylureido derivative of formula VII

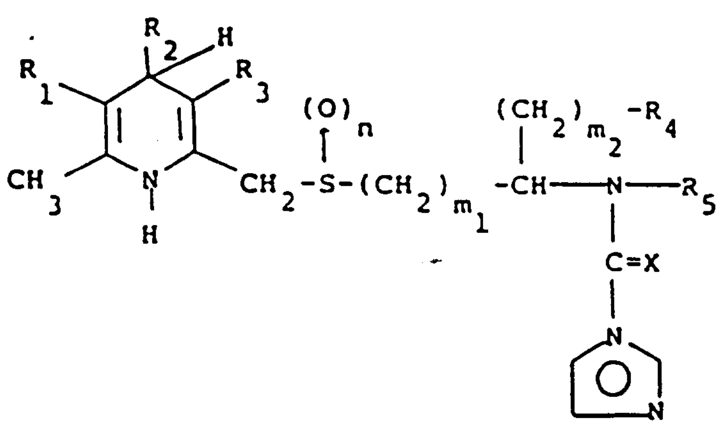

(VII)

wherein $R_1$, $R_2$, $R_3$, $R_4$, $R_5$, n, X, $m_1$ and $m_2$ have the above described meanings.

Compounds VII are then reacted with an alcohol, a thiol or an amine of formula VI.

For the acylation reaction of a compound of formula II with a diimidazolide V, equimolecular amounts or a slight excess of the acylating agent are used, the imidazolyl derivative VII can be isolated or reacted "in situ" with equimolecular amounts of a slight excess of the compounds of formula VI. The preferred conditions for the reaction (temperature, solvent, time) are substantially the same as those above described for substantially similar synthetic transformations, the only difference being a difference sequence of addition of the reagents.

The compounds II are described in WO-A-87/00836

The compounds II wherein $m_1$ is 2, 3, 4, $m_2$ is 1, $R_4$ is hydrogen and $R_5$ is hydrogen, $C_1$-$C_4$-alkyl or $COCF_3$ are described in EP-A-0 200 524.

The compounds of formulae IV, V and VI are easily available or they may be prepared from known compounds according to known methods.

The compound of the invention may be used in human and veterinary therapy for the treatment of hypertensive diseases of different origin and of coronary diseases.

In order to show the utility of the compounds as antihypertensive agents, they have been tested in genetically hypertensive rats studying, after oral administration, both the antihypertensive effect after a single administration (acute treatment) and after once a day administration repeated for 5 consecutive days.

In acute experiments, the antihypertensive effect of the compounds of the invention is dose-related in a dose range from 0.05 mg/kg to 3.5 mg/kg. Repeated administration shows that low dosages are effective to mantain, in the long time, mean blood pressure at the curated levels.

Specific examples of compounds of the invention tested in these experiments are the following: 3-ethyl-5-methyl-2-(4-amino-2'-thiabutyl)-4-(m-nitrophenyl)-6-methyl-3,5-dicarboxy-1,4-dihydropyridine; its diethylester: 3,5-diethyl-2-(4-butylamino-2'thiabutyl)-4-(m-nitrophenyl)-6-methyl-3,5-dicarboxy-1,4-dihydropyridine in comparison with their carbonylamino derivatives: aminocarbonyl, aminothiocarbonyl, 4'-methylpiperazinocarbonyl and ethoxycarbonyl derivatives.

The compounds of formula I induce a long lasting antihypertensive effect with a slow onset of the antihypertensive action. A slow and progressive decrease of the blood pressure is generally observed, the maximum effect being recorded after a period not shorter than 3 hours and ranging from 3 to 6-8 hours according to the administered dose. The decrease of the blood pressure may vary from 10 to 80-100 mmHg, depending from the dose but surprisingly, the antihypertensive effect does not seem to be coupled with reflex tachycardia.

Sometimes, an unexpected bradycardic effect is noticed also after treatment with relatively small dosages and this effect is also long-lasting.

The efficacy of low dosage treatment regimen is proved by repeated administration experiments: the persistance of the antihypertensive effect coupled with a progressive adjustment of the mean systolic pressure and heart-rate values to costantly and progressively decreasing values was observed.

Pressure and heart rate values were lower than basal ones at the end of each day of treatment.

In view of said features, the compounds of the invention are particularly useful for treating antihypertensive conditions, because of the slow and progressive onset of action, allowing single daily administrations, without significant modification of heart-rate.

The compounds of the invention may be administered by various routes to achieve the desired effect. The compounds may be administered to patient in pure form or as pharmaceutical compositions, by the oral or parenteral route.

The formulation of suitable pharmaceutical compositions may be carried out according to known techniques, as described for instance in "Remington's Pharmaceutical Sciences Handbook", Hack Publishing Co., U.S.A.

For the oral administration the compounds may be prepared in solid or liquid preparations in form of capsules, pills, tablets, powders, solutions, suspensions or emulsions. The solid unit dose can be a hard or soft gelatine capsule, containing lubricants or inert excipients such as lactose, saccharose or starch.

The compounds of the invention can also be formulated in form of tablets using the conventional excipients such as lactose, saccharose, starch, gelatine, alginic acid, stearic acid, magnesium stearate, etc.

For the parenteral administration the compounds of the invention may be administered as injectable solutions dissolved or suspended in physiologically acceptable diluents, with a sterile vehicle such as water or an oil, with or without addition of other excipients. The oils used can be of vegetal, animal, mineral or synthetic origin, such as peanut, soya or mineral oil. Generally, as a vehicle for injectable solutions, it is possible to use water, aqueous solutions of mineral salts, aqueous solutions of dextrose or other sugars, ethanol, glycols, such as propylene or polyethylene glycols.

The compounds of the invention may also be administered by the rectal route as suppositories, mixed with conventional vehicles such as for example cocoa butter, wax, polyvinylpyrrolidone or polyoxyethylenglycole and derivatives thereof.

The preferred administration route is the oral one.

The following examples illustrate but do not limit the invention.

Abbreviations EtOH, THF, MeOH, $Et_2O$, AcOEt refer respectively to ethanol, tetrahydrofurane, methanol, ethyl ether and ethyl acetate.

## EXAMPLE 1

A solution of of 2-(2-aminoethylthiomethyl)-3,5-dicarboethoxy-4-(m-nitrophenyl)-6-methyl-1,4-dihydropyridine hydrochloride (3 g) in AcOEt (30 ml) is treated under stirring with a solution of urea (0.8 g) in MeOH (8 ml).

The solvent is evaporated under reduced pressure. The glassy solid so obtained is heated to 140 °C for 2.5 hours, then cooled to room temperature, diluted with AcOEt (38 ml), washed with a sodium dihydrogen phosphate saturated solution (1 x 10 ml) and then with water (2 x 10 ml), dried on $Na_2SO_4$ and the solvent is evaporated under reduced pressure.

The reaction mixture is purified by chromatography on $SiO_2$(60 g eluent $CHCl_3$/MeOH-9.5/0.5).

2.8 g of 2-[2-(N-ureidyl)ethylthiomethyl]-3,5-dicarboethoxy-4-(m-nitrophenyl)-6-methyl-1,4-dihydropyridine; amorphous solid.

NMR ($\delta$ , $CDCl_3$): 1.20 (t, 6H); 2.35 (s, 3H); 2.50-3.05 (m, 2H); 3.45 (m, 2H); 4.10 (m, 6H); 5.15 (m, 3H): 5.90 (m, 1H); 7.20-8.15 (m, 5H).

## EXAMPLE 2

A solution of 2-(2-aminoethylthiomethyl)-3,5-dicarboethoxy-4-(m-nitrophenyl)-6-methyl-1,4-dihydropyridine (3 g) kept at 0 °C in anhydrous THF (30 ml) is treated with a solution of carbonyldiimidazole (1.34 g ) in anhydrous THF (16 ml) under nitrogen atmosphere.

The reaction mixture is warmed to room temperature and concentrated under reduced pressure, the residue is diluted with water and ice and then extracted with AcOEt (40 ml); the organic phase (AcOEt) is separated, washed with $H_2O$ (2 x 10 ml), dried on $Na_2SO_4$ and the solvent is evaporated under reduced pressure.

3.5 g of 2-[2-(imidazol-1-yl-carbamoyl)ethylthiomethyl]-3,5-dicarboethoxy-4-(m-mitrophenyl)-6-methyl-1,4-dihydropyridine are obtained as an amorphous solid.

NMR ($\delta$ , $CDCl_3$): 1.20 (t, 6H); 2.35 (s, 3H); 2.80 (m, 2H); 3.45-3.80 (m, 3H); 3.85-4.30 (m, 6H); 5.10 (s,1H): 6.90-8.30 (m, 8H).

## EXAMPLE 3

A solution of 2-[2-(imidazol-1-yl-carbamoyl)ethylthiomethyl]-3,5-dicarboethoxy-4-(m-nitrophenyl)-6-methyl-1,4-dihydropyridine (3.5 g) in THF (35 ml) is treated at 0 °C under nitrogen atmosphere with a solution of N-(2-hydroxyethyl)piperazine (0.9 g) in THF (10 ml).

The mixture is refluxed for 3 hours and then it is concentrated at reduced pressure, poured in $H_2O$ and extracted with AcOEt (40 ml); the AcOEt phase is separated and the residue is washed with $H_2O$ (2 x 5 ml) and dried on $Na_2SO_4$. The solvent is eliminated by distillation at reduced pressure.

The residue is purified by chromatography on $SiO_2$ (90 g eluent $CHCl_3$/MeOH 9/1).

3.4 g of 2-2-[4-(2-hydroxyethyl)-piperazin-1-yl-carbonyl-amino]-ethylthiomethyl-3,5-dicarboethoxy-4-(m-nitrophenyl)-6-methyl-1,4-dihydropyridine are obtained as an amorphous solid.

NMR ($\delta$ , $CDCl_3$): 1.20 (t, 6H); 2.25-2.90 (m, 12H); 3.20-3.75 (m, 8H); 3.80-4.35 (m, 6H); 5.10 (s, 1H); 5.35 (m, 1H): 7.20-8.15 (m, 5H).

Using in the experimental precedure of example 3 an amine selected in the group of aqueous ammonia, N-methylpiperazine, butylamine and methylamine and a dihydropyridine selected in the group of:

- 2-[2-(imidazol-1-yl-carbamoyl)ethylthiomethyl]-3,5-dicarboethoxy-4-(m-nitrophenyl)-6-methyl-1,4-dihydropyridine;

the following compounds are prepared:

- 2-[2-(3-methylureidyl)ethylthiomethyl]-3,5-dicarboethoxy-4-(m-nitrophenyl)-6-methyl-1,4-dihydropyridine,amorphous solid;

- 2-[2-(3-butylureidyl)ethylthiomethyl]-3,5-dicarboethoxy-4-(m-nitrophenyl)-6-methyl-1,4-dihydropyridine.
  NMR (δ , $CDCl_3$): 0.80-1.55 (m, 13H); 2.40 (s, 3H); 2.75 (m, 2H); 2.90-3.50 (m, 5H); 3.80-4.25 (m, 6H); 5.10 (s, 1H); 5.40 (m, 1H); 7.20-8.15 (m, 5H).
- 2-{2-[4-methylpiperazin-1-yl-carbamoyl]-ethylthiomethyl}-3,5-dicarboethoxy-4-(m-nitrophenyl)-6-methyl-1,4-dihydropyridine;
  NMR (δ , $CDCl_3$): 1.20(t, 6H); 2.15-2.90 (m, 10H); 3.10-3.85 (m, 8H); 3.90-4.35 (m, 6H); 5.10 (s,1H); 5.55 (m,1H); 7.20-8.15 (m,5H).

**EXAMPLE 4**

A solution of methylisothiocyanate (0.63 g) in dimethoxyethane (6 ml) is added to a solution of 2-(2-aminoethylthio)methyl-3,5-dicarboethoxy-4-(m-nitrophenyl)-6-methyl-1,4-dihydropyridine (2.7 g). After 3,5 hours, the reaction mixture is concentrated to small volume, poured in $H_2O$/ice and extracted with AcOEt (30 ml). The AcOEt phase is washed with a $NaHCO_3$ saturated solution (1 x 5 ml), $Na_2HPO_4$ saturated solution (1 x 5 ml) and water, dried on $Na_2SO_4$ and the solvent is evaporated under vacuum.

The crude residue is purified by chromatography on $SiO_2$ (60 g, eluent $CNCl_3$/AcOEt 9/1).

2.5 g of 2-[2-(2-methylthioureidyl)ethylthiomethyl]-3,5-dicarboethoxy-4-(m-nitrophenyl)-6-methyl-1,4-dihydropyridine, amorphous solid, are obtained.

$^1$H-NMR (δ , $CDCl_3$): 1.20 (t, 6H); 2.35 (s, 3H); 2.60-3.10 (m, 5H); 3.50-4.35 (m, 8H); 5.10 (s, 1H); 6.35-6.70 (m, 2H); 7.20-8.15 (m, 5H).

Using this procedure, the phenylisothiocyanate, 2-[2-(2-phenylthioureidyl)ethylthiomethyl]-3,5-dicarboethoxy-4-(m-nitrophenyl)-6-methyl-1,4-dihydropyridine is obtained.

**EXAMPLE 5**

A solution of ethylchloroformate (0.65 ml) in 1,2-dichloroethane (3 ml) is added to a solution of 2-(2-aminoethylthiomethyl)-4-(m-nitrophenyl)-3,5-dicarboethoxy-6-methyl-1,4-dihydropyridine (3 g) and triethylamine (0.93 ml) in 1,2-dichloroethane (30 ml) at 0 °C. The reaction mixture is warmed to room temperature and diluted with $H_2O$ (10 ml); the organic phase (1,2-dichloroethane) is separated and washed with $H_2O$ (1 x 5 ml); dried on $Na_2SO_4$ and evaporated to dryness under a reduced pressure.

The crude residue is purified by chromatography on $SiO_2$ (60 g, eluent CHCl/AcOEt 7/3).

3.5 g of 2-(2-ethoxycarbamoylethylthiomethyl)-3,5-dicarboethoxy-4-(m-nitrophenyl)-6-methyl-1,4-dihydropyridine are obtained, as an oil.

NMR (δ , $CDCl_3$): 1.20 (t, 9H); 2.40 (s, 3H); 2.80 (m, 2H); 3.15-3.70 (m, 2H); 3.80-3.45 (m, 8H); 5.10 (s, 1H): 5.50 (m, 1H); 7.20-8.15 (m, 5H).

**EXAMPLE 6**

A solution of p-toluensulphonylchloride (1.56 g) in $CH_2Cl_2$ (15 ml) is added dropwise to a solution of 2-(2-aminoethylthiomethyl)-3-carboethoxy-5-carbomethoxy-4-(m-nitrophenyl)-6-methyl-1,4-dihydropyridine (3.5 g) and triethylamine (1.2 ml) in $CH_2Cl_2$ (35 ml), maintaining the temperature at 0 °C.

The solution is warmed to room temperature and after 30' it is treated with a $NaHCO_3$ saturated solution (10 ml). The organic phase is separated, washed with water (2 x 10 ml), dried on $Na_2SO_4$ and evaporated under reduced pressure.

The residue is triturated in boiling $Et_2O$, thus obtaining 3.8 g of 2-(2-p-toluensulphonamidoethylthiomethyl)-3-carboethoxy-5-carbomethoxy-4-(m-nitrophenyl)-6-methyl-1,4-dihydropyridine, m.p. 136-138 °C.

Using in this procedure benzenesulphonylchloride or methanesulphonylchloride, the following compounds were obtained:

- 2-(2-benzenesulphonamidoethylthiomethyl)-3-carboethoxy-5-carbomethoxy-4-(m-nitrophenyl)-6-methyl-1,4-dihydropyridine;
- 2-(2-methanesulphonamidoethylthiomethyl)-3-carboethoxy-5-carbomethoxy-4-(m-nitrophenyl)-6-methyl-1,4-dihydropyridine.

## Claims

**1.** Compounds of formula I

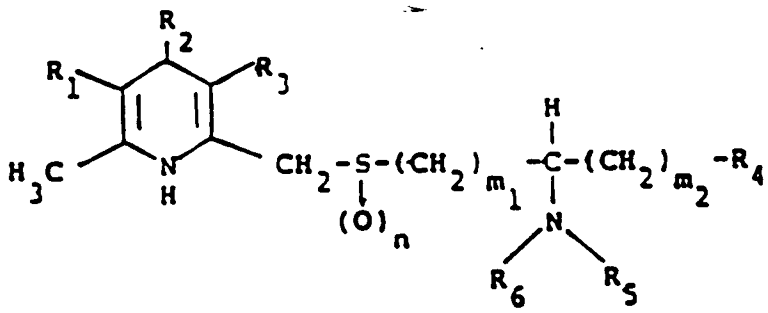

(I)

wherein

- $R_1$ is a C1-C3-alkoxycarbonyl group
- $R_2$ is a m-nitrophenyl group;
- $R_3$ is a $COOR_7$ group;
- $R_4$ is hydrogen or methyl;
- $R_5$ is hydrogen or butyl;
- $R_6$ is a

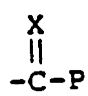 or 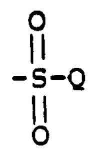

group
wherein X is oxygen or sulphur; P is $C_1$-$C_5$-alkylthio, benzylthio, $C_1$-$C_5$-alkoxy, benzyloxy group and an amino group

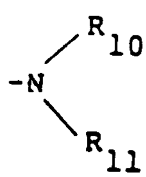

wherein $R_{10}$ and $R_{11}$, that can be the same or different, are hydrogen, $C_1$-$C_5$-alkyl or, taken together with the nitrogen atom, form a piperazine, 4-methyl-piperazine, or 4-(2-hydrxyethyl)-piperazine ring; Q is $C_1$-$C_5$-alkyl, phenyl or benzyl;

- $R_7$ is a $C_1$-$C_6$ alkyl group,
- n is 0, 1 or 2; $m_1$ is 1 or 2 and $m_2$ is 0 or 1
their optical isomers, diastereoisomers and pharmaceutically acceptable salts.

**2.** Compounds according claim 1, wherein $R_6$ is an aminocarbonyl group.

**3.** Compounds according to Claim 1, wherein $R_6$ is an aminothiocarbonyl group.

**4.** Compounds according to claim 1, wherein $R_6$ is an $C_1$-$C_5$-alkoxy carbonyl or $C_1$-$C_5$-alkylthiocarbonyl group.

**5.** Compounds according to claim 1, wherein $R_6$ is a phenyl- or benzylsulphonyl group.

**6.** Compounds according to claim 1
2-[2-(N-ureidyl)ethylthio]methyl-6-methyl-3,5-dicarboethoxy-4(3-nitro-phenyl)-1,4-dihydropyridine,
2-[2-(/4-(2-hydroxyethyl)piperazin-1-yl/carbonylamino)ethylthio]methyl-6-methyl-3,5-dicarboethoxy-4(3-nitro-phenyl)-1,4-dihydropyridine,
2-[2-(4-methylpiperazin-1-yl-carbamoyl)ethylthio)methyl-6-methyl-3,5-dicarboethoxy-4(3-nitro-phenyl)-1,4-dihydropyridine,
2-[2-(2-methylthioureidyl)ethylthio]methyl-6-methyl-3,5-dicarboethoxy-4(3-nitro-phenyl)-1,4-dihydropyridine,
2-[2-(ethoxycarbamoyl)ethylthio]methyl-6-methyl-3,5-dicarboethoxy-4(3-nitro-phenyl)-1,4-dihydropyridine
2-[2-(3-butylureidyl)ethylthio]methyl-6-methyl-3,5-dicarboethoxy-4(3-nitro-phenyl)-1,4-dihydropyridine.

**7.** 2-[2-(p-toluensulfonylamino)ethylthio]methyl-6-methyl-3,5-dicarboethoxy-4(3-nitro-phenyl)-1,4-dihydropyridine.

**8.** A process for the preparation of compounds of formula I, characterized in that a compound of formula II

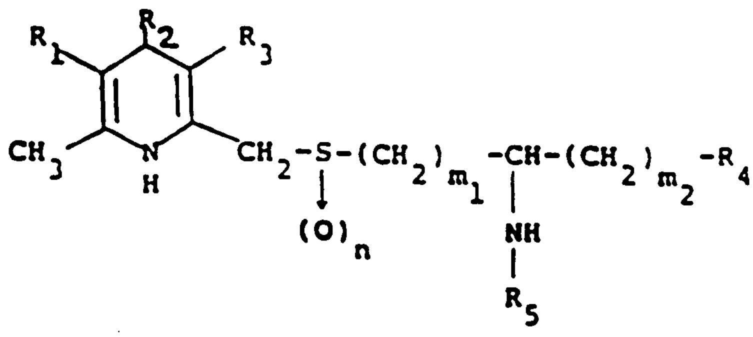

(II)

wherein $R_1$, $R_2$, $R_3$, $R_4$, $R_5$, n, $m_1$, $m_2$ are as above defined with an acylating agent as an activated form of an ester or an amide of a carbonic or thiocarbonic acid of formula III or an activated form of a sulphonic acid of formula IV

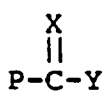

(III)

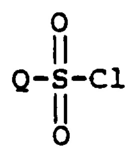

(IV)

wherein P, Q and X are as above defined and Y is selected in the group consisting of chlorine and 1-imidazolyl, in the presence or not of a base to give a compound of formula I.

**9.** Pharmaceutical compositions containing as an active principle a compound of claims from 1 to 7, mixtured with a pharmaceutically acceptable vehicle.

## Patentansprüche

**1.** Verbindungen der Formel I

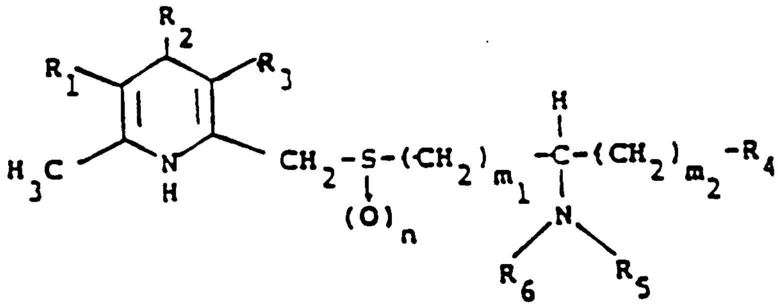

(I)

wobei

- $R_1$ für eine C1-C3-Alkoxycarbonylgruppe
- $R_2$ für eine m-Nitrophenylgruppe;
- $R_3$ für eine $COOR_7$-Gruppe;
- $R_4$ für Wasserstoff oder Methyl;
- $R_5$ für Wasserstoff oder Butyl;
- $R_6$ für eine

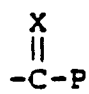 oder 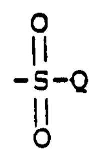

Gruppe steht
wobei X für Sauerstoff oder Schwefel steht; P für eine $C_1$-$C_5$-Alkylthiogruppe, eine Benzylthiogruppe, eine $C_1$-$C_5$-Alkoxygruppe, eine Benzyloxygruppe und eine Amino-Gruppe

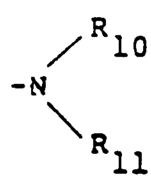

steht, wobei $R_{10}$ und $R_{11}$, die gleich oder unterschiedlich sein können, für eine Wasserstoffgruppe oder eine $C_1$-$C_5$-Alkylgruppe stehen oder zusammen mit dem Stickstoffatom einen Piperazin-, einen 4-Methylpiperazin oder einen 4-(2-Hydroxyethyl)piperazin-Ring bilden; Q für eine $C_1$-$C_5$-Alkyl-, Phenyl- oder Benzylgruppe steht;
- $R_7$ für eine $C_1$-$C_6$-Alkylgruppe steht,
- n 0,1 oder 2; $m_1$ 1 oder 2 und $m_2$ 0 oder 1 bedeuten,

ihre optischen Isomere , Diastereoisomere und pharmazeutisch geeignete Salze.

**2.** Verbindungen gemäß Anspruch 1, wobei $R_6$ für eine Aminocarbonylgruppe steht.

**3.** Verbindungen gemäß Anspruch 1, wobei $R_6$ für eine Aminothiocarbonylgruppe steht.

**4.** Verbindungen gemäß Anspruch 1, wobei $R_6$ für eine $C_1$-$C_5$-Alkoxycarbonyl- oder $C_1$-$C_5$-Alkylthiocarbonylgruppe steht.

5. Verbindungen gemäß Anspruch 1, wobei $R_6$ für eine Phenyl- oder Benzylsulfonylgruppe steht.

6. Verbindungen gemäß Anspruch 1
2-[2-(N-Ureidyl)ethylthio]methyl-6-Methyl-3,5-Dicarboethoxy-4(3-Nitrophenyl)-1,4-Dihydropyridin,
2-[2-(/4-(2-Hydroxyethyl]piperazin-1-yl/carbonylamino)ethylthio]methyl-6-Methyl-3,5-Dicarboethoxy-4(3-Nitrophenyl)-1,4-Dihydropyridin,
2-[2-(4-Methylpiperazin-1-yl-carbamoyl)ethylthio]methyl-6-Methyl-3,5-Dicarboethoxy-4(3-Nitrophenyl)-1,4-Dihydropyridin,
2-[2-(2-Methylthioureidyl)ethylthio]methyl-6-Methyl-3,5-Dicarboethoxy-4(3-Nitrophenyl)-1,4-Dihydropyridin,
2-[2-(ethoxycarbamoyl)ethylthio]methyl-6-Methyl-3,5-Dicarboethoxy-4(3-Nitrophenyl)-1,4-Dihydropyridin,
2-[2-(3-butylureidyl)ethylthio]methyl-6-Methyl-3,5-Dicarboethoxy-4(3-Nitrophenyl)-1,4-Dihydropyridin,

7. 2-[2-(p-toluolsulfonylamino)ethylthio]methyl-6-Methyl-3,5-Dicarboethoxy-4(3-Nitrophenyl)-1,4-Dihydropyridin.

8. Ein Verfahren zur Herstellung von Verbindungen der Formel I, dadurch gekennzeichnet, daß man eine Verbindung der Formel II

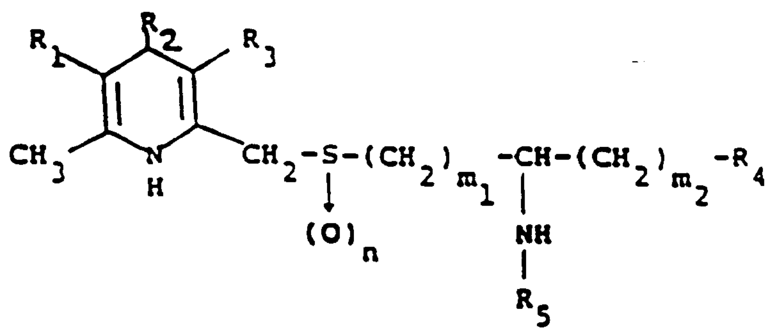

(II)

wobei $R_1$, $R_2$, $R_3$, $R_4$, $R_5$, n, $m_1$, $m_2$, wie oben definiert sind, mit einem acylierenden Agens als aktivierte Form eines Esters oder eines Amids einer Carbon- oder Thiocarbonsäure der Formel III oder als aktivierte Form einer Sulfonsäure der Formel IV umsetzt

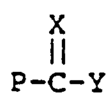

(III)

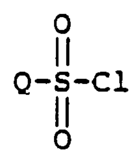

(IV)

wobei P, Q und X wie oben definiert sind und Y aus der Gruppe bestehend aus Chlor und 1-Imidazolyl ausgewählt wird, und so in Gegenwart oder Abwesenheit einer Base eine Verbindung der Formel I entsteht.

9. Pharmazeutische Zubereitungen, die als einen wirksamen Bestandteil eine Verbindung der Ansprüche 1 bis 7 enthalten und denen ein pharmazeutisch geeignetes Vehikel beigemischt ist.

## Revendications

**1.** Composés de formule I

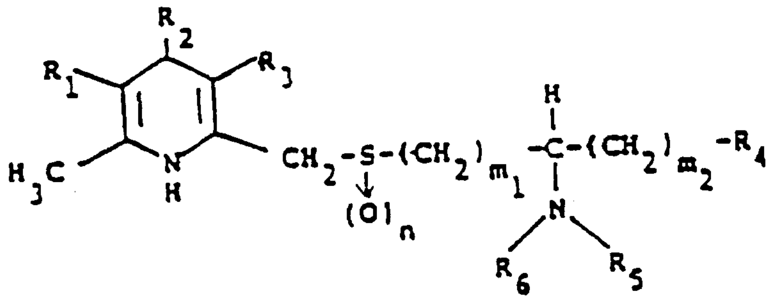

(I)

dans laquelle

- $R_1$ représente un groupe alkoxycarbonyle en $C_1$ à $C_3$;
- $R_2$ représente un groupe m-nitrophényle;
- $R_3$ représente un groupe $COOR_7$;
- $R_4$ représente un atome d'hydrogène ou un groupe méthyle;
- $R_5$ représente un atome d'hydrogène ou un groupe butyle;
- $R_6$ représente un groupe

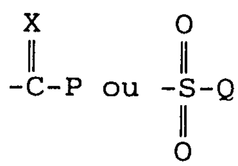

dans lequel X représente un atome d'oxygène ou de soufre; P représente un groupe alkylthio en $C_1$ à $C_5$, benzylthio, alkoxy en $C_1$ à $C_5$, benzyloxy et un groupe amino

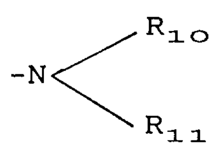

dans lequel $R_{10}$ et $R_{11}$, qui peuvent être identiques ou différents, représentent un atome d'hydrogène, un groupe alkyle en $C_1$ à $C_5$ ou, pris conjointement avec l'atome d'azote, forment un noyau pipérazine, 4-méthyl-pipérazine, ou 4-(2-hydroxyéthyl)pipérazine; Q représente un groupe alkyle en $C_1$ à $C_5$, phényle ou benzyle;
- $R_7$ représente un groupe alkyle en $C_1$ à $C_6$,
- n vaut 0, 1 ou 2; $m_1$ vaut 1 ou 2 et $m_2$ vaut 0 ou 1,

leurs isomères optiques, diastéréoisomères et sels acceptables sur le plan pharmaceutique.

**2.** Composés selon la revendication 1, dans lesquels $R_6$ représente un groupe aminocarbonyle.

**3.** Composés selon la revendication 1, dans lesquels $R_6$ représente un groupe aminothiocarbonyle.

**4.** Composés selon la revendication 1, dans lesquels $R_6$ représente un groupe alkoxy(en $C_1$ à $C_5$)-carbonyle ou alkyl(en $C_1$ à $C_5$)thiocarbonyle.

**5.** Composés selon la revendication 1, dans lesquels $R_6$ représente un groupe phényle ou benzylsulphonyle.

**6.** Composés selon la revendication 1,
2-[2-(N-uréidyl)éthylthio]méthyl-6-méthyl-3,5-dicarboéthoxy-4(3-nitro-phényl)-1,4-dihydropyridine,
2-[2-(/4-(2-hydroxyéthyl)pipérazin-1-yl/carbonylamino)éthylthio]méthyl-6-méthyl-3,5-dicarboéthoxy-4(3-nitrophényl)-1,4-dihydropyridine,
2-[2-(4-méthylpipérazine-1-yl-carbamoyl)éthylthio]méthyl-6-méthyl-3,5-dicarboéthoxy-4(3-nitro-phényl)-1,4-dihydropyridine,
2-[2-(2-méthylthiouréidyl)éthylthio]méthyl-6-méthyl-3,5-dicarboéthoxy-4(3-nitro-phényl)-1,4-dihydropyridine,
2-[2-(éthoxycarbamoyl)éthylthio]méthyl-6-méthyl-3,5-dicarboéthoxy-4(3-nitro-phényl)-1,4-dihydropyridine,
2-[2-(3-butyluréidyl)éthylthio]méthyl-6-méthyl-3,5-dicarboéthoxy-4(3-nitro-phényl)-1,4-dihydropyridine.

**7.** 2-[2-(p-toluènesulfonylamino)éthylthio]méthyl-6-méthyl-3,5-dicarboéthoxy-4(3-nitro-phényl)-1,4-dihydropyridine.

**8.** Procédé pour la préparation des composés de formule I, caractérisé en ce qu'un composé de formule II

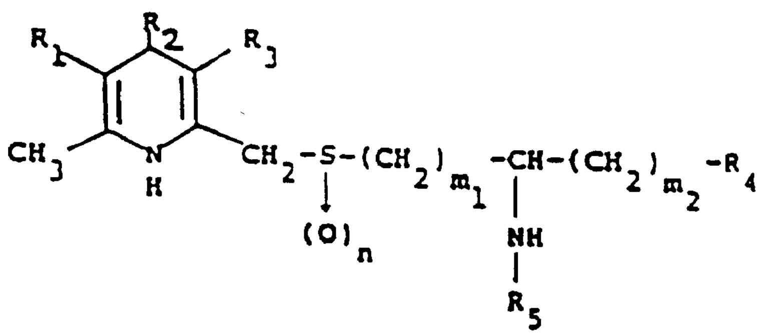

(II)

dans laquelle $R_1$, $R_2$, $R_3$, $R_4$, $R_5$, n, $m_1$, $m_2$ sont tels que définis ci-dessus réagit avec un agent acylant sous forme activée d'un ester ou d'un amide d'un acide carbonique ou thiocarbonique de formule III ou sous forme activée d'un acide sulfonique de formule IV

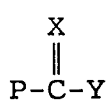

(III)

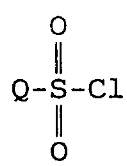

(IV)

dans laquelle P, Q et X sont tels que définis ci-dessus et Y est choisi dans le groupe constitué du chlore et du 1-imidazolyle, en présence ou non d'une base pour obtenir un composé de formule I.

**9.** Compositions pharmaceutiques contenant comme principe actif un composé des revendications 1 à 7, mélangé avec un véhicule acceptable sur le plan pharmaceutique.